# EUROPÄISCHE PATENTSCHRIFT

(11) **EP 0 577 799 B1**
(45) Veröffentlichungstag und Bekanntmachung des Hinweises auf die Patenterteilung: **22.07.1998**
(21) Anmeldenummer: 93902069.9
(22) Anmeldetag: 28.01.1993
(51) Int. Cl.: A61B 10/00, A61B 5/103, B60J 1/00, G01N 31/22

(54) **INDIKATOR ZUR ERMITTLUNG UND ANZEIGE DES FETT- UND FEUCHTIGKEITSGEHALTES MENSCHLICHER HAUT**
INDICATOR FOR DETERMINING AND INDICATING THE FAT AND MOISTURE CONTENT OF HUMAN SKIN
INDICATEUR DE DETERMINATION ET D'INDICATION DU TAUX DE GRAISSE ET D'HYDRATATION DE L'EPIDERME

(30) Priorität: 28.01.1992 DE 4202277; 27.01.1993 DE 4302218
(43) Veröffentlichungstag der Anmeldung: 12.01.1994
(73) Patentinhaber: BREHM, Robert, D-82467 Garmisch Partenkirchen (DE)
(72) Erfinder: BREHM, Robert, D-8100 Garmisch Partenkirchen (DE); PUGH, Robert, Richmond, Surrey TWG 2EL (GB)
(74) Vertreter: Preissner, Nicolaus, Dipl.-Ing.
(86) Internationale Anmeldenummer: DE9300080
(87) Internationale Veröffentlichungsnummer: WO9314699

(56) Entgegenhaltungen:
- EP-A- 0 179 419
- EP-A- 0 282 823
- WO-A-84/02460
- DE-A- 2 106 408
- US-A- 4 313 393
- US-A- 4 488 547
- US-A- 5 094 248
- SCHMIDT M.: 'Anorganische Chemie', Band 1, Seite 269

## Beschreibung

Die Erfindung bezieht sich auf einen Indikator zur Ermittlung und Anzeige des Fett- und Feuchtigkeitsgehaltes menschlicher Haut, mit einer auf einem Träger aufgebrachten fettempfindlichen Substanz, die nach dem Aufbringen auf die Haut zumindest partiell den physikalischen Brechungsindex ändert.

Bekanntlich wird insbesondere die Gesichtshaut durch eine dünne Fettschicht und austretenden Schweiß als Säureschutzmantel geschützt. Dazu sind im Gesicht besonders viele Talgdrüsen vorhanden, die Fett in Form von Tag produzieren, wobei der Tag über Ausführungsgänge an die Oberfläche der Haut gelangt und als fettiger Film insbesondere einen Witterungsschutz bildet.

Größere Talgdrüsen befinden sich dabei vorzugsweise in der sogenannten T-Zone des Gesichtes mit der Stirn als Querbalken und der Nase bis zum Kinn als senkrechter Strich. Hier sieht man häufiger fettigglänzende Zonen, während die Wangenpartien in der Regel matter erscheinen, da hier die Talgdrüsen sehr viel kleiner sind.

Je nach Hauttyp variiert selbstverständlich das Erscheinungsbild der Haut bzw. die einzelnen Gesichtsregionen sind unterschiedlich "fettig" durch eine hohe Talgprodukion oder "trocken" durch mangelnden Wassergehalt in der obersten Hautschicht. Es ist manchmal schwer zu differenzieren, ob bei einer trockenen, spröde erscheinenden Haut ein Defizit an Fett oder Feuchtigkeit vorliegt. Ein Mangel an Feuchtigkeit kann dabei immer, d.h. sowohl bei fettigem Hauttyp als auch bei trockenem Hauttyp vorliegen, während ein Mangel an Fett in der Regel nur bei trockenem Hauttyp vorliegt, so daß demnach eine Feuchtigkeitszufuhr in den meisten Fällen erforderlich ist.

Aus kosmetischer Sicht ist es dabei von Bedeutung, den Hautzustand an vielen Stellen zu ermitteln und Hautpräparate auszuwählen, die für den entsprechenden Hautzustand anwendbar sind, um einen Schaden oder eine Verschlechterung des Hautzustandes zu verhüten.

Zur Ermittlung des Hautzustandes ist es dabei beispielsweise aus der US-PS 45 32 937 bekannt, einen dünnen offenzellligen mikroporösen und hydrophoben Film aus einem Polymermaterial mittels eines talgdurchlässigen Klebers auf die Haut aufzubringen und dort eine Weile zu belassen, wobei sich die Poren des Filmes mit Talg füllen und dadurch transparent werden, so daß damit eine erste Abschätzung des Talggehaltes ermöglicht wird. Nachteilig bei diesem Verfahren ist jedoch, daß eine Einwirkzeit von mindestens 30 Minuten erforderlich ist und daß diese Methode nicht für detaillierte Studen und zur längeren Aufbewahrung- des Untersuchungsergebnisses geeignet ist, da der in den Poren aufgenommene Talg dazu neigt, sich sehr schnell über den gesamten Film zu verteilen.

Weiterhin ist aus der DE-OS 21 06 408 eine Testfolie zum Ermitteln des Fettstatus der Haut bekannt, die eine weiße Deckschicht aus feindispersem Kunststoff aufweist. Bei Auflegen der Folie auf die Haut erhält man auf der weißen Beschichtung ein unregelmäßiges Muster schwarzer Punkte, die den Talgdrüsen der Haut entsprechen, so daß der Fettgehalt der Haut sichtbar gemacht wird.

Der vorliegenden Erfindung liegt daher die Aufgabe zugrunde, einen Indikator anzugeben, mit dem auf einfache Weise und insbesondere innerhalb sehr kurzer Zeit der Fett- und/oder Feuchtigkeitszustand der Haut ermittelt und angezeigt werden kann.

Zur Lösung dieser Aufgabe ist erfindungsgemäß vorgesehen, daß die Substanz als fett- und feuchtigkeitsempfindliches, hochporöses und angenähert weißfarbiges Siliciumdioxid vorliegt, das auf einen Träger aufgebracht ist, der eine von weiß abweichende Farbgebung aufweist, wobei das Siliciumdioxid abhängig vom Fett- und Feuchtigkeitsgehalt der Haut seinen physikalischen Brechungsindex derart ändert, daß der darunterliegende Träger zumindest partiell durchscheint und sichtbar wird.

Bei einem derartigen Indikator wird also eine schnell ansprechende, fett- und feuchtigkeitsempfindliche Substanz verwendet, die bei Kontakt mit Wasser oder Fett aufgrund einer rein physikalischen Reaktion ihren Brechungsindex ändert, so daß dadurch die darunterliegende Farbschicht sichtbar wird.

Zweckmäßig ist es dabei, wenn der Träger eine größere Fläche als die aufgebrachte Substanz aufweist und die Substanzfläche allseitig mit gleicher Breite überragt.

Dabei kann der Träger aus Papier oder Kunststoff bestehen, wobei der Träger auf seiner Rückseite noch mit einer selbstklebenden Lage beschichtet sein kann, die mit einem Schutzpapier abgedeckt ist.

Die Substanz selbst kann mit bekannten Druckverfahren auf den Träger aufgedruckt werden, wobei die Schichtdicke der Substanz 10 bis 50 µ, insbesondere 20 bis 30 µ, betragen soll. Für die Anwendung gibt es zwei verschiedene, besonders zweckmäßige Möglichkeiten. Einmal können mehrere gleich oder unterschiedlich geformte Indikatoren aus Träger und aufgedruckter Substanz von einer jeweiligen Größe von etwa 5 bis 10 cm² auf einer schematischen, ebenen Gesichtsmaske an den zu messenden Stellen abnehmbar aufgeheftet sein.

Es ist aber auch möglich und besonders zweckmäßig, wenn auf eine, einer schematischen Gesichtsmaske entsprechenden flexiblen Folie an den zu messenden Stellen Trägerbereiche aufgedruckt und mit der Substanz abgedeckt sind. Damit kann diese Gesichtsmaske als Ganzes auf das Gesicht aufgepreßt werden.

Zweckmäßig ist es, wenn diese bedruckte Gesichtsmaske mit einem Schutzblatt abgedeckt ist und Gesichtsmaske und Schutzblatt seitliche über eine Perforation abtrennbare Ansätze aufweisen, die miteinander verklebt sind, um damit Gesichtsmaske und Schutzblatt miteinander zu verbinden.

Von besonderem Vorteil ist es, wenn die Gesichtsmaske einen Vergleichsmaßstab für unterschiedliche Fett- und Feuchtigkeitsgehalte aufweist.

Anhand einer schematischen Zeichnung sind Aufbau und Funktion von Ausführungsbeispielen nach der Erfindung näher erläutert. Dabei zeigen:
- Fig. 1: den Schichtaufbau eines solchen Indikators für Einzelanwendungen im Längsschnitt,
- Fig. 2: eine Aufsicht auf einen Indikator im frischen, unbenutzten Zustand,
- Fig. 3: eine Aufsicht auf einen solchen Indikator nach Abnehmen von den entsprechenden Hautpartien,
- Fig. 4: eine schematisierte Gesichtsmaske mit aufgeklebten Indikatoren und Vergleichsmaßstab und
- Fig. 5: eine flexible Gesichtsmaske mit aufgedrucktem, von der Substanz abgedeckten Trägerbereich.

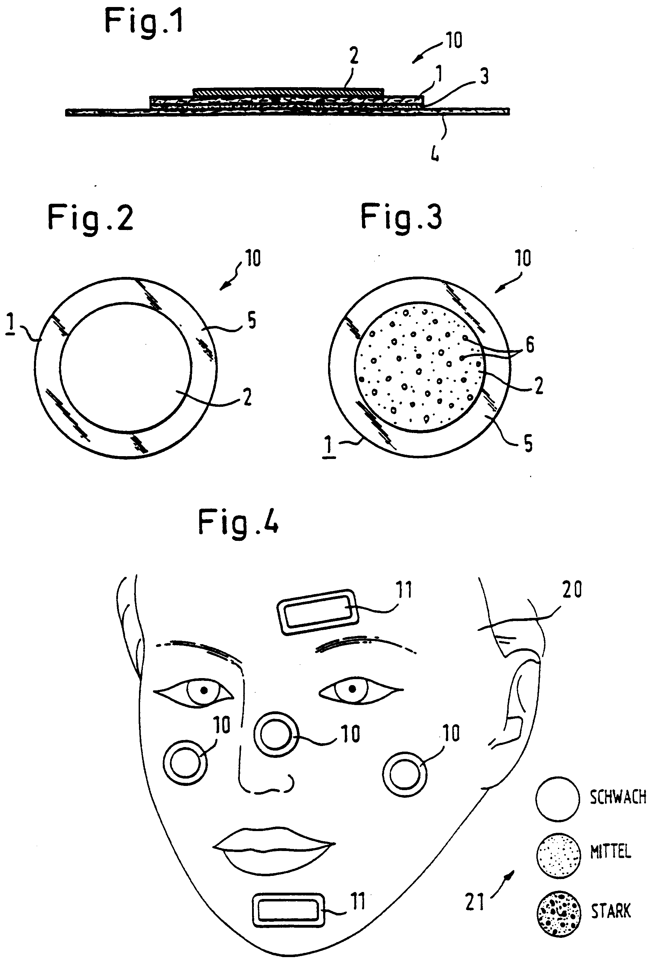

Wie man aus dem Schnitt im stark vergrößerten Maßstab nach Fig. 1 ersieht, ist auf einen Träger 1, der aus Papier, PVC oder Polyester bestehen kann und zweckmäßigerweise eine farbige Oberfläche aufweist, im geringeren Umfang eine Substanz 2 aufgetragen, die die Eigenschaft hat, aufgrund einer farbigen Reaktion ihren Brechungsindex bei in Berührung kommen mit Feuchtigkeit oder Fett zu ändern, so daß dadurch die farbige Beschichtung des Trägers 1 nach außen durchscheint. Diese Substanz 2 ist zweckmäßigerweise in einem Druckvorgang auf den Träger aufgebracht und zwar in herkömmlichen Techniken, wie Offset-Druck, Lithodruck, Rollbeschichtung oder Siebdruck oder in jeder bekannten anderen Art und Weise.

Im vorliegenden Fall ist der Träger 1 mit der Substanz 2 im Siebdruckverfahren mit einer Stärke von 15 bis 50 µ, vorzugsweise 20 bis 30 µ, beschichtet, die für die gewünschte Wirkungsweise voll ausreichend ist.

Bei einer Einzelanwendung eines solchen Indikators 10, der aus diesem Träger 1 und der Substanz 2 besteht, ist es zweckmäßig, wenn der Träger 1 auf seiner Rückseite noch mit einer selbstklebenden Lage 3 beschichtet ist, um ihn damit - wie noch später erläutert wird - vor seiner Anwendung zunächst auf eine größere Fläche aufzukleben oder mit einem Schutzpapier 4 abzudecken.

Wie man aus Fig. 2 ersieht, kann ein solcher einzelner Indikator 10 beispielsweise als runde Scheibe gefertigt oder nachträglich ausgestanzt werden, wobei der Träger 1 zweckmäßigerweise die eigentliche Substanzschicht 2 im Durchmesser überragt und damit einen unbeschichteten Rand 5 bildet. Für einen guten Kontrast kann dabei der Träger 1 z.B. blau eingefärbt oder bedruckt sein, während die Substanz 2 zweckmäßigerweise weiß ist.
Ein solcher Indikator 10 wird dann mit der Substanzseite 2 auf die entsprechenden Hautpartien aufgedruckt und kann schon nach etwa 15 Sekunden wieder entfernt werden. Dabei haben dann entsprechend Fig. 3 entsprechende Bereiche 6 der Substanz 2, die mit besonders fetthaltigen Partien oder Talgdrüsen in Berührung gekommen sind, ihren Brechungsindex dahingehend geändert, daß die an sich weiße Substanz 2 in diesen Bereichen durchsichtig geworden ist, so daß damit der blaue Hintergrund des Trägers 1 sichtbar wird. Je nach Größe und Anzahl dieser durchsichtigen Bereiche 6 und des farbigen Erscheinungsbildes kann daraus auf einen hohen oder geringen Fettgehalt oder Feuchtigkeitsgehalt der Haut geschlossen werden.

Selbstverständlich ist es auch möglich, die Substanzschicht 2 in einem lichten Farbton einzufärben, wobei jedoch berücksichtigt werden muß, daß sich der Brechungsindex der Substanz nicht zu stark durch den Zusatz der entsprechenden Farbpigmente ändert oder vermindert.

Eine mögliche Anwendung zur Erleichterung der Ermittlung der Hauteigenschaften ist in Fig. 4 dargestellt. Dabei sind auf eine schematische Gesichtsmaske 20 an den Stellen, die zweckmäßigerweise für eine Beurteilung des Hautzustandes herangezogen werden, bereits entsprechende Indikatoren aufgeklebt und zwar beispielsweise runde Indikatoren 10 auf den Wangen und auf der Nase und rechteckige Indikatoren 11 im Stirn- und Kinnbereich. Für die Feststellung des Fett- und Feuchtigkeitsgehaltes der Haut werden dann diese auf die Masken geklebten Indikatoren 10 bzw. 11 von der Maske 20 abgenommen, auf die entsprechenden Hautpartien des Gesichtes aufgedrückt und anschließend nach der beschriebenen kurzen Einwirkungszeit von etwa 15 Sekunden zurück auf die Maske geklebt. In einem seitlich von der Maske angebrachten Maßstab 21 kann dann unmittelbar im Vergleich mit den zurückgeklebten Indikatoren auf den Fettgehalt der Haut geschlossen werden. Dabei ist die Verfärbung der an sich weißen Substanz 2 umso größer, je fetter die Haut ist, so daß dann entsprechende Gegenmaßnahmen eingeleitet oder entsprechende Präparate ausgewählt werden können.

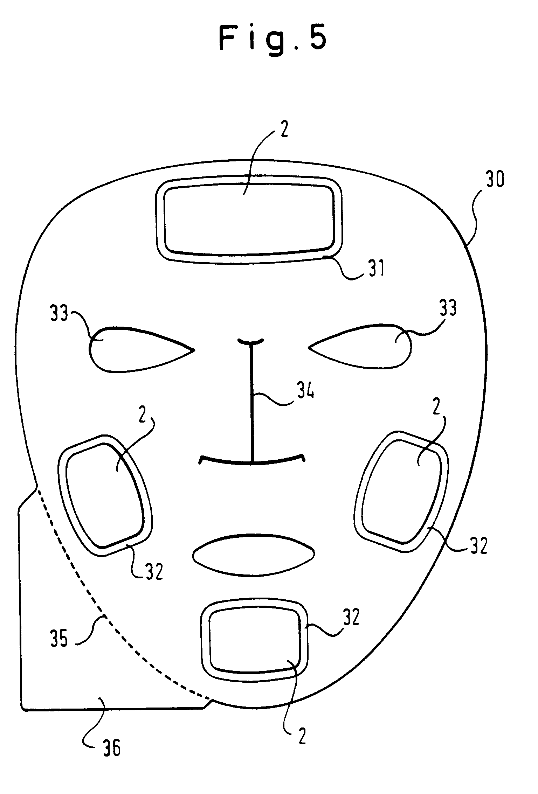

Eine weitere Anwendungsmöglichkeit ist in Fig. 5 dargestellt. Dabei sind auf eine schematische Gesichtsmaske 30 aus Papier oder einer flexiblen Folie bereits an den zu messenden Stellen Trägerbereiche 31 bzw. 32 in kleinerer Ausführung aufgedruckt, die dann mit der entsprechenden Substanz 2 abgedeckt sind. Ferner weist diese Maske 30 Ausschnitte 33 für die Augen sowie eingeprägte Schlitze 34 für die Nase auf, so daß dann diese Maske 30 unmittelbar auf das Gesicht aufgedrückt werden kann, dort eine Weile verbleibt, wobei nach dem Abnehmen dann die entsprechende Verfärbung der Substanz 2 festgestellt und mit einem Maßstab verglichen werden kann.

Zur leichteren Handhabung ist es zweckmäßig, wenn diese Maske 30 mit einem - nicht näher dargestellten - Deckblatt gleicher Außenkontur abgedeckt ist. Dazu weisen die Gesichtsmaske 30 und das Deckblatt zweckmäßigerweise seitliche über eine Perforation 35 abtrennbare Ansätze 36 auf, die miteinander verklebt sind, so daß damit Schutzblatt und Maske 30 miteinander verbunden sind. Zum Gebrauch wird dann die überstehende Ecke 36 abgetrennt, so daß allein die Maske 30 handhabbar und auf das Gesicht aufdrückbar ist.

Insgesamt ergibt sich also ein Indikator, mit dem auf schnelle und einfache Weise Fett- und/oder Feuchtigkeitsgehalt der Haut ermittelt werden können.

## Patentansprüche

1. Indikator zur Ermittlung und Anzeige des Fettgehaltes menschlicher Haut, mit einer auf einem Träger (1; 31, 32) aufgebrachten fettempfindlichen Substanz (2), die nach Aufbringen auf die Haut zumindest partiell den physikalischen Brechungsindex ändert, dadurch gekennzeichnet, daß die Substanz (2) als fett- und feuchtigkeitsempfindliches, hochporöses und angenähert weißfarbiges Siliciumdioxid vorliegt, das auf einem Träger (1; 31, 32) aufgebracht ist, der eine von weiß abweichende Farbgebung aufweist, wobei das Siliciumdioxid abhängig vom Fett- und Feuchtigkeitsgehalt der Haut seinen physikalischen Brechungsindex derart ändert, daß der darunterliegende Träger (1; 31, 32) zumindest partiell durchscheint und sichtbar wird.

2. Indikator nach Anspruch 1, dadurch gekennzeichnet, daß der Träger (1) eine größere Fläche als die aufgebrachte Substanz (2) aufweist und die Substanzfläche allseitig mit gleicher Breite überragt.

3. Indikator nach Anspruch 1, dadurch gekennzeichnet, daß der Träger (1) aus Papier oder Kunststoff besteht.

4. Indikator nach Anspruch 2, dadurch gekennzeichnet, daß der Träger (1) auf seiner Rückseite mit einer selbstklebenden Lage (3) beschichtet ist, die mit einem Schutzpapier (4) abgedeckt ist.

5. Indikator nach einem der Ansprüche 1 bis 4, dadurch gekennzeichnet, daß die Substanz (2) auf den Träger (1; 31, 32) aufgedruckt ist.

6. Indikator nach Anspruch 5, dadurch gekennzeichnet, daß die Substanz (2) eine Schichtdicke von 15 bis 50 µ, insbesondere 20 bis 30 µ, aufweist.

7. Indikator nach Anspruch 5 oder 6, dadurch gekennzeichnet, daß jeder aus Träger (1) und aufgedruckter Substanz (2) bestehende Indikator (10) eine Größe von etwa 5 bis 10 cm² aufweist.

8. Indikator nach Anspruch 7, dadurch gekennzeichnet, daß mehrere, gleich oder unterschiedlich geformte Indikatoren (10, 11) auf einer schematischen, ebenen Gesichtsmaske (20) an den zu messenden Stellen abnehmbar angeheftet sind.

9. Indikator nach einem oder mehreren der Ansprüche 1 bis 7, dadurch gekennzeichnet, daß auf eine, einer schematischen Gesichtsmaske (30) entsprechenden flexiblen Folie an den zu messenden Stellen Trägerbereiche (31, 32) aufgedruckt und mit der Substanz (2) abgedeckt sind.

10. Indikator nach Anspruch 8 oder 9, dadurch gekennzeichnet, daß die bedruckte Gesichtsmaske (20, 30) mit einem Schutzblatt abgedeckt ist.

11. Indikator nach Anspruch 10, dadurch gekennzeichnet, daß Gesichtsmaske (30) und Schutzblatt seitliche, über eine Perforation (35) abtrennbare Ansätze (36) aufweisen, die miteinander verklebt sind.

12. Indikator nach Anspruch 8 oder 9, dadurch gekennzeichnet, daß die Gesichtsmaske (20, 30) einen Vergleichsmaßstab (21) für unterschiedliche Fett- und Feuchtigkeitsgehalte aufweist.

## Claims

1. An indicator for determining and indicating the fat content of human skin comprising applied to a substrate (1; 31, 32) a fat-sensitive substance (2) changing the physical refractive index at least in part after being applied to the skin, characterized in that said substance (2) exists as fat and moisture sensitive, highly porous and near white-colored silicium dioxide which is applied to a substrate (1; 31, 32) featuring a coloration other than white, said silicium dioxide changing its physical refractive index in response to said skin fat and moisture content such that said substrate (1; 31, 32) located thereunder shows through at least in part and becomes visible.

2. The indicator as set forth in claim 1, characterized in that said substrate (1) comprises a larger surface area than that of said applied substance (2) and exceeds said substance surface area on all sides by the same amount in width.

3. The indicator as set forth in claim 1, characterized in that said substrate (1) consists of a paper or plastics material.

4. The indicator as set forth in claim 2, characterized in that said substrate (1) is coated on its reverse side with a self-adhesive layer (3) which is covered by a protective paper (4).

5. The indicator as set forth in any of the claims 1 to 4, characterized in that said substance (2) is impressed on said substrate (1; 31, 32).

6. The indicator as set forth in claim 5, characterized in that said substance (2) has a film thickness of 15 to 50 µ, more particularly 20 to 30 µ.

7. The indicator as set forth in claim 5 or 6, characterized in that each indicator (10) comprising a substrate (1) and impressed substance (2) is approx. 5 to 10 cm² in size.

8. The indicator as set forth in claim 7, characterized in that several, identically or differingly shaped indicators (10, 11) are removably attached to a schematic, flat face mask (20) at sensing locations.

9. The indicator as set forth in any of the claims 1 to 7, characterized in that substrate zones (31, 32) are impressed on a flexible film corresponding to a schematic face mask (20) at said sensing locations and are covered by said substance (2).

10. The indicator as set forth in claim 8 or 9, characterized in that said impressed face mask (20, 30) is covered by a protective sheet.

11. The indicator as set forth in claim 10, characterized in that face mask (30) and protective sheet comprise side tabs (36) bonded to each other, removable via a perforation (35) .

12. The indicator as set forth in claim 8 or 9, characterized in that said face mask (20, 30) comprises a comparison scale (21) for differing fat and moisture contents.

## Revendications

1. Indicateur pour détecter et afficher la teneur en graisse de l'épiderme, comportant une substance (2) sensible à la graisse et déposée sur un substrat (1 ; 31, 32), laquelle modifie, après déposition sur l'épiderme, du moins partiellement son indice de réfraction physique, caractérisé en ce que la substance (2) se présente sous forme de dioxyde de silicium sensible à la graisse et à l'humidité, hautement poreux et approximativement de couleur blanche, lequel est déposé sur un substrat (1 ; 31, 32) qui présente une coloration différente de la couleur blanche, le dioxyde de silicium modifiant son indice de réfraction en dépendance de la teneur en graisse et en humidité de l'épiderme, de telle sorte que le substrat sous-jacent (1 ; 31, 32) devient du moins partiellement apparent et visible.

2. Indicateur selon la revendication 1, caractérisé en ce que le substrat (1) présente une surface plus grande que la substance déposée (2) et dépasse de tous les côtés de la même largeur au-delà de la surface de la substance.

3. Indicateur selon la revendication 1, caractérisé en ce que le substrat (1) est constitué en papier ou en matière plastique.

4. Indicateur selon la revendication 2, caractérisé en ce que le substrat (1) est revêtu sur sa face arrière d'une couche autocollante (3) qui est recouverte d'un papier protecteur (4).

5. Indicateur selon l'une quelconque des revendications 1 à 4, caractérisé en ce que la substance (2) est imprimée sur le substrat (1 ; 31, 32).

6. Indicateur selon la revendication 5, caractérisé en ce que la substance (2) présente une épaisseur de couche de 15 à 50 µ, en particulier de 20 à 30 µ.

7. Indicateur selon l'une ou l'autre des revendications 5 et 6, caractérisé en ce que chaque indicateur (10) constitué d'un substrat (1) et d'une substance imprimée (2) présente une taille d'environ 5 à 10 cm².

8. Indicateur selon la revendication 7, caractérisé en ce que plusieurs indicateurs (10, 11) de formes identiques ou différentes sont appliqués de façon détachable aux emplacements à mesurer sur un masque de visage schématique plat (20).

9. Indicateur selon l'une ou plusieurs des revendications 1 à 7, caractérisé en ce que des régions de substrat (31, 32) sont imprimées aux emplacements à mesurer sur un film flexible correspondant à un masque de visage schématique (30), et en ce qu'elles sont recouvertes avec la substance (2).

10. Indicateur selon l'une ou l'autre des revendications 8 et 9, caractérisé en ce que le masque de visage imprimé (20, 30) est recouvert avec une feuille protectrice.

11. Indicateur selon la revendication 10, caractérisé en ce que le masque de visage (30) et la feuille protectrice présentent des pattes latérales (36) détachables via une perforation (35), qui sont collées les unes avec les autres.

12. Indicateur selon l'une ou l'autre des revendications 8 et 9, caractérisé en ce que le masque de visage (20, 30) présente une échelle de comparaison (21) pour différentes teneurs en graisse et en humidité.
